# EUROPEAN PATENT APPLICATION

(11) **EP 4 763 091 A1**
(43) Date of publication of application: **24.06.2026**
(21) Application number: 24221397.3
(22) Date of filing: 19.12.2024
(51) Int. Cl.: A61B 6/10, A61B 6/46

(54) **A CONNECTOR ASSEMBLY FOR ATTACHING A USER INTERFACE TO A RADIATION SHIELD**

(71) Applicant: Koninklijke Philips N.V., 5656 AE Eindhoven (NL)
(72) Inventor: CHITTA, Sudeendra, Eindhoven (NL); ARENDS, Marc Victor, Eindhoven (NL); CONTINI, Enrico, 5656AG Eindhoven (NL)
(74) Representative: Philips Intellectual Property & Standards

(57) **Abstract**

A connector assembly for attaching a user interface to the radiation shield for use with a medical imaging system. The connector assembly (103) enables a user to move the user interface relative to the radiation shield (101). The connector assembly comprises a first part (104), a second part (105) and a third part (106). A first end of the first part is hingedly connected to a first end of the second part (105) and a second end of the first part is arranged for coupling to the radiation shield. A first end of the third part is slidably coupled with a second end of the second part and the second end of the third part is arranged to be coupled to the user interface (102).

## Description

### FIELD OF THE INVENTION

The invention relates to a connector assembly for attaching a user interface to a radiation shield. The invention further relates to a radiation shield assembly comprising the radiation shield,. The invention further relates to a medical imaging system comprising the radiation shield assembly.

### BACKGROUND OF THE INVENTION

An interventional procedure needs the minimum dose needed to complete the procedure successfully. The focus in a cardiac catheter laboratory (or cathlab) is to balance between image quality and radiation dose. The aim is to perform a diagnostic and treatment procedure successfully whilst minimizing the dose to the patient and the physician. Radiation exposure is a significant risk for physicians, and therefore radiation management is of key importance. To mitigate this risk, radiation shields are commonly used in medical X-ray systems. These shields are designed to protect physicians during an intervention from harmful ionizing radiation. It is an object of the invention to further improve the radiation protection while allowing them to perform their duties during an intervention.

US10945687B2 discloses a transparent radiation shield, attachable to a patient support platform, and movable to shield a physician from imaging radiation, includes a transparent computer display that is controllable to provide a data overlay on the shield pertaining to patient data and/or x-ray images. US10945687B2 does not disclose a connector assembly and therefore does provide limited flexibility for the physician.

### SUMMARY OF THE INVENTION

The object of the invention is achieved with connector assembly of claim 1. Advantageous embodiments are defined in the dependent claims.

An aspect of the invention discloses a connector assembly for attaching a user interface to a radiation shield. The connector assembly is configured to enable a user (e.g. a physician) to move the user interface relative to the radiation shield. The connector assembly comprises a first part, a second part, and a third part. A first end of the first part being hingedly connected to a first end of the second part, a second end of the first part being arranged for coupling to the radiation shield. A first end of the third part being slidably coupled with a second end of the second part, the second end of the third part being arranged to be coupled to the user interface. The connector assembly provides the advantage of enabling attaching the user interface to the radiation shield. The radiation shield provides a comprehensive shielding solution that safeguards the user from radiation exposure during medical imaging procedures while enabling the user to move the user interface relative to the radiation shield, thus providing flexibility. This enables the physician to benefit from the protection of the radiation shield while providing input on the user interface or reading information on the user interface. A further advantage of the connector assembly is that with the first, second and third part, the angle and distance of the user interface towards the physician can be adjusted to enhance ergonomic positioning for the physician during an intervention procedure and with the second end of the first part of the connector assembly being arranged to couple to the existing radiation shield present in the cathlabs, the operating space in cathlabs is not occupied by the addition of another shielding equipment. Yet another advantage is that the added cost for benefitting from the radiation shielding is low.

In an embodiment, the second part of the connector assembly has a main axis and a hollow portion extending along the main axis and the third part is arranged to slide in the hollow portion, The second part further comprises a locking mechanism configured to lock in position the third part. This configuration provides the advantage of allowing precise and secure positioning of the user interface relative to the radiation shield. The sliding mechanism allows for precise adjustments of the user interface relative to the radiation shield. The locking feature ensures that once the desired position is achieved, the user interface remains stable and secure. The ability to easily adjust and lock the user interface enhances usability during medical imaging procedures. It ensures that the user interface is positioned optimally for the user, improving ergonomics, and protecting the user from radiation exposure.

In an embodiment, the connector assembly comprises a socket for providing an electrical coupling to the user interface. This provides the advantage of improved reliability as the battery of the user interface may be exhausted, especially when a series of medical procedures is planned during a day.

In an embodiment, the invention discloses a radiation shield assembly for a medical imaging system. The radiation shield assembly comprises the radiation shield, the user interface and the connector assembly. This integration provides the advantage of a comprehensive shielding solution that safeguards the user from radiation exposure during medical imaging procedures and enables the user to move the user interface relative to the radiation shield.

In an embodiment, the radiation shield comprises a mount, and the second end of the first part is configured to attach the connector assembly to the mount. This arrangement ensures a secure and stable connection between the radiation shield and the user interface.

In an embodiment, the radiation shield comprises a boom for coupling the radiation shield to a ceiling and the mount being rotatably coupled to the boom. This configuration provides the advantage of greater flexibility and ease of movement of the radiation shield, making it more convenient for the user to position and adjust the plane of radiation shield as needed, thus increasing the protection from radiation exposure.

In an embodiment, the mount has a cross section, and the second end of the first part has a hole matching the cross section to attach the connector assembly to the mount. This configuration provides the advantage of ensuring a secure and stable attachment of the connector assembly to the mount.

In an embodiment, the second end of the first part is coupled to the mount via a gripping mechanism. This configuration provides the advantage of ensuring a secure and stable attachment of the connector assembly to the radiation shield.

In an embodiment, the radiation shield comprises a bracket and the bracket is coupled to the mount. This configuration provides the advantage of enhancing the stability and support of the radiation shield to the mount, ensuring that it remains securely attached and properly positioned during use of medical procedures.

In an embodiment, the user interface comprises a touch screen module providing virtual buttons for controlling the medical imaging system. This configuration provides the advantage of enhancing control and usability by allowing operators to interact with the system through an intuitive and user-friendly touch screen interface. The virtual buttons provide easy access to various functions and settings, improving the overall efficiency and effectiveness of medical imaging procedures.

The present invention also provides a medical imaging system comprising an imaging assembly and the radiation shield assembly. This arrangement provides the advantage of integrating the radiation shield assembly with the imaging assembly, ensuring that the radiation shield is always positioned correctly relative to the imaging assembly. This integration enhances the overall safety and efficiency of the medical imaging system, as it ensures that the radiation shield is always in place to protect the operator from ionizing radiation during imaging procedures.

These and other aspects of the invention will be apparent from and elucidated with reference to the embodiments described hereinafter.

### BRIEF DESCRIPTION OF THE DRAWINGS

Fig. 1 illustrates a connector assembly for attaching a user interface to a radiation shield in accordance with an embodiment of the invention.
Fig. 2 illustrates a side view of the connector assembly mentioned in Fig. 1.
Fig. 3 illustrates an example of a medical imaging system comprising an imaging assembly and the radiation shield assembly.

### DESCRIPTION OF EMBODIMENTS

Certain embodiments are described in greater detail with reference to the accompanying drawings. In the following description, similar drawing reference numerals are used for similar elements, even in different drawings. The details provided in the description, such as construction and elements, are intended to assist in a comprehensive understanding of the exemplary embodiments. Well-known functions or constructions are not described in detail to avoid obscuring the embodiments with unnecessary information.

Unless otherwise noted, when an element or component is said to be "connected to," "coupled to," another element or component, it will be understood that the element or component can be directly connected or coupled to the other element or component, or intervening elements or components may be present. That is, these and similar terms encompass cases where one or more intermediate elements or components may be employed to connect two elements or components. However, when an element or component is said to be "directly connected" to another element or component, this encompasses only cases where the two elements or components are connected to each other without any intermediate or intervening elements or components.

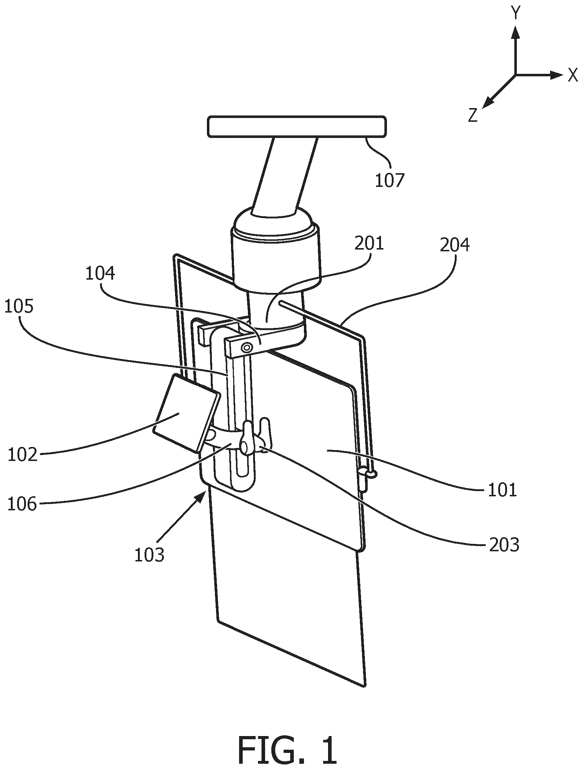

Fig. 1 illustrates a connector assembly 103 for attaching a user interface 102 to a radiation shield 101 in accordance with an embodiment of the invention. The connector assembly being configured to enable a user to move the user interface relative to the radiation shield 101. The connector assembly comprises a first part 104, a second part 105 and a third part 106. A first end of the first part 104 being hingedly connected to a first end of the second part 105, a second end of the first part 104 being arranged for coupling to the radiation shield 101. A first end of the third part 106 being slidably coupled with a second end of the second part, the second end of the third part being arranged to be coupled to the user interface 102.

The first end of the first part is hingedly connected to the first end of the second part. The hinged connection can be achieved through various means, including mechanical hinges (pin hinges, continuous hinges), flexible hinges (elastomeric hinges), bolted joints and ball-and-socket joints. These configurations allow for various movements, such as rotation, flexing, and tilting, providing flexibility in positioning the user interface relative to radiation shield. The second end of the first part is arranged for coupling to the radiation shield, which can be achieved through bolting mechanisms, clamps, magnetic connectors, and snap-fit connectors.

The connector assembly also comprises a third part, with the first end of the third part being slidably coupled with the second end of the second part. The sliding connection can be achieved through sliding rails and grooved tracks. The second end of the third part is arranged to be coupled to the user interface, which can be achieved through VESA mount standards, bolting mechanisms, clamps, and magnetic connectors.

The materials used for the connector assembly can include metals (aluminum, steel, titanium), plastics (polycarbonate, ABS), and composites (carbon fiber, fiberglass).

The user interface can be adjusted vertically, allowing it to move up or down relative to the radiation shield. Further, the user interface can also be tilted at various angles, providing flexibility in positioning and improving ergonomics during medical imaging procedures. When the desired configuration of the user interface 102 is obtained, the user interface may be locked in place to have a stable connection. An example of a user interface is the Philips Azurion ^{™} touch screen module, see for example Philips Touch screen module pro | Image-guided therapy systems.

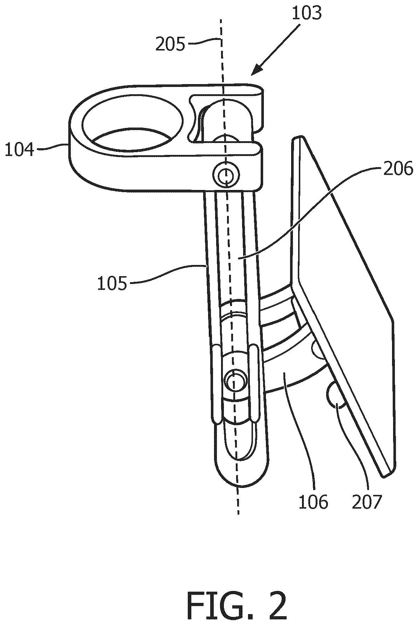

Fig. 2 illustrates a side view of the connector assembly mentioned in Fig. 1. In an embodiment, the second part 105 of the connector assembly has a main axis 205 and a hollow portion 206 extending along the main axis. The third part 106 is arranged to slide in the hollow portion. The sliding connection can be achieved through various means, including telescoping mechanisms, sliding rails, and grooved tracks. The second part 105 further comprises a locking mechanism 203 configured to lock the third part 106 in position. The locking mechanism can include mechanical locks, friction locks, or magnetic locks. This configuration provides the advantage of allowing precise and secure positioning of the user interface relative to the radiation shield. The sliding mechanism allows for precise adjustments of the user interface relative to the radiation shield. The locking feature ensures that once the desired position is achieved, the user interface remains stable and secure. The ability to easily adjust and lock the user interface enhances usability during medical imaging procedures. It ensures that the user interface is positioned optimally for the user, improving ergonomics and protecting the user from radiation exposure.

In an embodiment, the connector assembly further comprises a socket 207 for providing an electrical coupling to the user interface. The socket 207 can be configured to provide power, data transfer, or both. The electrical coupling can be achieved through various means, including wired connections (e.g., USB, HDMI, power cables) and wireless connections (e.g., Bluetooth, Wi-Fi). For wireless connections, a battery (not shown) can also be an option. The materials used for the socket can include metals (e.g., copper, aluminum) for conductive parts and plastics (e.g., polycarbonate, ABS) for insulating parts. The electrical coupling also allows for data transfer between the user interface and other components of the medical imaging system, enhancing the overall functionality and usability of the system.

In an embodiment, the invention discloses a radiation shield assembly for a medical imaging system 300. The radiation shield assembly is placed within a procedure room that comprises the radiation shield 101, the user interface 102, and the connector assembly 103. The radiation shield protects the user from ionizing radiation during medical imaging procedures. The user interface is operatively coupled to the connector assembly, enabling the user to move the user interface relative to the radiation shield. The materials used for the radiation shield can include lead acrylic glass, metal, or transparent polymeric sheets coated with radiation-absorbing material, such as lead or non-toxic alternatives like tungsten or barium sulfate. This ensures that the radiation shield provides effective protection while maintaining visibility for the user. In an embodiment, the radiation shielding material(s) is transparent so that placement of the radiation shield for maximizing protection from radiation does not obstruct the visual line of sight of the user. Alternatively, the transparent radiation shielding material may include transparent polymeric sheets coated with radiation absorbing material.

In an embodiment, the radiation shield assembly further comprises a mount 201. The second end of the first part 104 is configured to attach the connector assembly to the mount 201 in a manner that enables movement of the first part 104 relative to the mount 201 around at least one of the y-axis or the x-axis, indicated in Fig. 1, where the y-axis is the vertical axis and the x- axis is the horizontal axis. The attachment can be achieved through various means, including bolting mechanisms, clamps, magnetic connectors, and snap-fit connectors.

In an embodiment, the radiation shield assembly further comprises a boom 107 for coupling the radiation shield 101 to a ceiling and/or one or more walls of the procedure room (not shown). The mount 201 is rotatably coupled to the boom. The mount 201 is coupled to the second end of the first part (104) and/or the boom 107 in a manner that enables rotation of the first part 104 relative to the boom 107 around at least one of the y-axis, the x-axis or the z-axis, indicated in Fig. 1. The first part 104 can spin around the y-axis, tilt left and right around the z-axis, and tilt front and back around the x-axis in any combination of movements. The mount 201 may include any compatible mounting hardware that enables movement in one or more directions. For example, the mount 201 may include a gimbal fixedly attached to the boom 107 and rotationally attached to the first part 104. Alternatively, the mount 201 may include a gimbal fixedly attached to the first part 104 and rotationally attached to the boom 107.The boom 107 can be made from materials such as aluminum, steel, or other suitable metals, ensuring durability and strength. The rotatable coupling can be achieved through various means, including mechanical hinges, ball bearings, or swivel joints, allowing for controlled rotation of the radiation shield.

In an embodiment, the mount 201 has a cross section, and the second end of the first part 104 has a hole matching the cross section to attach the connector assembly to the mount. The attachment can be achieved through various means, including bolting mechanisms, clamps, magnetic connectors, and snap-fit connectors.

In an embodiment, the second end of the first part 104 is coupled to the mount 201 via a gripping mechanism. The gripping mechanism can include mechanical grippers, magnetic grippers, snap-fit connectors, or clamp connectors.

In an embodiment, the radiation shield assembly further comprises a bracket 204. The bracket is coupled to the mount 201. The bracket may be connected via a thin rod that passes through the mount in order to attach the radiation shield to the mount.

In an embodiment, the user interface comprises a touch screen module (not shown) providing virtual buttons for controlling the medical imaging system. For example, in a radiology room, a user can use a touch screen to control the X-ray machine. The virtual buttons on the touch screen allow the technician to adjust parameters such as the intensity of the X-rays, the position of the patient, and other settings necessary to obtain high-quality images. This setup enhances usability and efficiency, as it allows the technician to make quick and precise adjustments without the need for physical controls. User interface may be a keyboard, joystick, track ball, stylus, or mouse.

The user interface 102 is operatively coupled to the connector assembly 103. The user interface 102 is coupled to the second end of the third part 106 of the connector assembly 103 in a manner that enables rotation of the user interface relative to the radiation shield 101 around at least one of the y-axis, the x-axis or the z-axis, indicated in Fig. 1, where the y-axis is the vertical axis and the x- axis and the z-axis are horizontal axes perpendicular to one another. The user interface 102 can spin around the y-axis, tilt left and right around the z-axis, and tilt front and back around the x- axis in any combinations of movements. The connector assembly may include any compatible mounting hardware that enables movement in one or more directions.

The user interface can be adjusted vertically, allowing it to move up or down relative to the radiation shield. Further, the user interface can also be tilted at various angles, providing flexibility in positioning and improving ergonomics during medical imaging procedures.

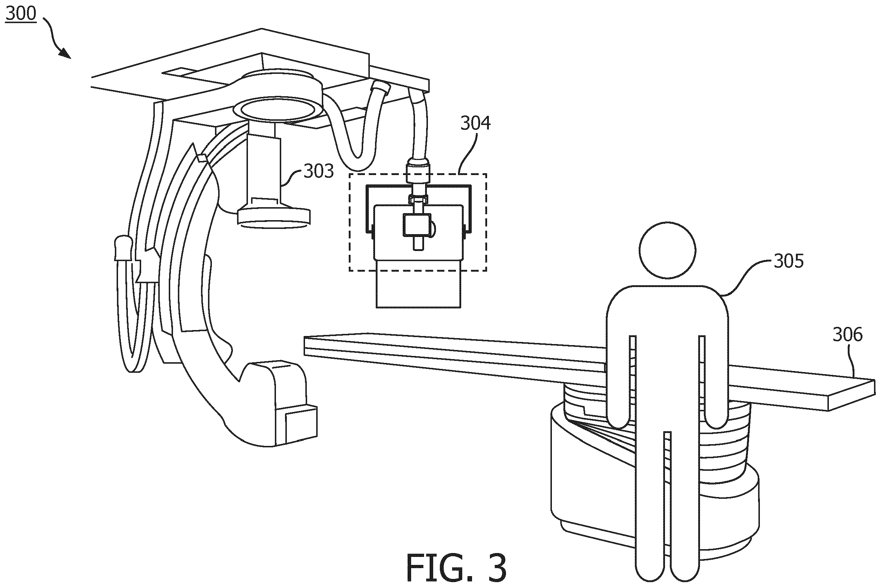

Fig. 3 illustrates an example of a medical imaging system 300. The system comprises an imaging assembly 303 and the radiation shield assembly 304. The imaging assembly 303 is configured to acquire an image (e.g. X-ray, CT) of a patient (not shown) lying on a table 306. The radiation shield assembly 304 comprises the radiation shield 101, the user interface 102 and the connector assembly 103 (shown in Fig. 1). The radiation shield is placed to protect users from ionizing radiation during medical imaging procedures. The user interface 102 allows user 305 to interact with the medical imaging system. The connector assembly (shown in Fig. 2) attaches the user interface to the radiation shield and enables the user 305 to move the user interface relative to the radiation shield. The user 305 may be surgeon, clinician or medical professional.

It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting the claim. The word "comprising" does not exclude the presence of elements or steps other than those listed in a claim. The word "a" or "an" preceding an element does not exclude the presence of a plurality of such elements. In the device claim enumerating several means, several of these means may be embodied by one and the same item of hardware. Measures recited in mutually different dependent claims may advantageously be used in combination.

## Claims

1. A connector assembly (103) for attaching a user interface (102) to a radiation shield (101), the connector assembly being configured to enable a user to move the user interface relative to the radiation shield (101), the connector assembly comprising
- a first part (104) and a second part (105), a first end of the first part being hingedly connected to a first end of the second part (105), a second end of the first part being arranged for coupling to the radiation shield;
- a third part (106), a first end of the third part being slidably coupled with a second end of the second part, the second end of the third part being arranged to be coupled to the user interface (102).

2. The connector assembly (103) according to claim 1 wherein the second part (105) has a main axis 205 and a hollow portion 206 extending along the main axis, wherein the third part (106) is arranged to slide in the hollow portion, and wherein the second part further comprises a locking mechanism (203) configured to lock in position the third part.

3. The connector assembly (103) according to claim 1 or 2 further comprising a socket (207) for providing an electrical coupling to the user interface (102).

4. A radiation shield assembly (304) for a medical imaging system (300), the radiation shield assembly comprising:
a radiation shield (101);
a user interface (102); and
the connector assembly of claim 1, 2 or 3.

5. The radiation shield assembly according to claim 4, wherein the radiation shield further comprises a mount (201), and the second end of the first part (104) is configured to attach the connector assembly to the mount (201).

6. The radiation shield assembly according to claim 5, wherein the radiation shield further comprises a boom (107) for coupling the radiation shield to a ceiling, the mount (201) being rotatably coupled to the boom.

7. The radiation shield assembly according to claim 5 or 6, wherein the mount (201) has a cross section and the second end of the first part (104) has a hole matching the cross section to attach the connector assembly to the mount (201).

8. The radiation shield assembly according to claim 5 or 6, wherein the second end of the first part (104) is coupled to the mount (201) via a gripping mechanism.

9. The radiation shield assembly according to any of the preceding claims 5 to 8, wherein the radiation shield (101) comprises a bracket (204), the bracket being coupled to the mount (201).

10. The radiation shield assembly according to any of the preceding claims 4 to 9, wherein the user interface comprises a touch screen module providing virtual buttons for controlling the medical imaging system.

11. A medical imaging system (300) comprising
an imaging assembly (303); and
the radiation shield assembly (304) as claimed in any of the preceding claims.
